# EUROPÄISCHE PATENTSCHRIFT

(11) **EP 0 531 716 B1**
(45) Veröffentlichungstag und Bekanntmachung des Hinweises auf die Patenterteilung: **23.12.1998**
(21) Anmeldenummer: 92113465.6
(22) Anmeldetag: 07.08.1992
(51) Int. Cl.: C12N 15/55, C12N 15/82, C12N 9/14

(54) **Deacetylasegene zur Erzeugung von Phosphinothricin oder Phosphinothricyl-Alanyl-Alanin, Verfahren zu ihrer Isolierung und ihre Verwendung**
Deacetylase gene for the production of phosphinothricin or phosphinothricyl-alanil-alanine, method of isolating it, and its use
Gène pour déacétylase, pour la production de phosphinothricine ou phosphinothricyl-alanil-alanine, procédé pour son isolation, et son utilisation

(30) Priorität: 09.08.1991 DE 4126414
(43) Veröffentlichungstag der Anmeldung: 17.03.1993
(60) Teilanmeldung: 98101772.6
(73) Patentinhaber: Hoechst Schering AgrEvo GmbH, 13509 Berlin (DE)
(72) Erfinder: Broer, Inge, Dr., W-4800 Bielefeld (DE); Hillemann, Doris, W-4800 Bielefeld (DE); Pühler, Alfred, Prof.Dr., W-4800 Bielefeld (DE); Wohlleben, Wolfgang, Dr., W-4800 Bielefeld (DE); Donn, Günter, Dr., W-6238 Hofheim/Taunus (DE); Müllner, Hubert, Dr., W-6233 Kelkheim (Taunus) (DE); Bartsch, Klaus, Dr., W-6374 Steinbach (DE)

(56) Entgegenhaltungen:
- WO-A-90/08828
- WO-A-91/03561
- GENE Bd. 63 , 1988 , AMSTERDAM NL Seiten 65 - 74 STRAUCH, E., ET AL. 'Cloning of a phosphinothricin N-acetyltransferase gene from Streptomyces viridochromogenes Tü494 and its expression in Streptomyces lividans and Escherichia coli'
- JOURNAL OF GENERAL MICROBIOLOGY Bd. 137, Nr. 2 , Februar 1991 Seiten 351 - 359 HARA, O., ET AL. 'The bialaphos biosynthetic genes of Streptomyces viridochromogenes: cloning, heterospecific expression, and comparison with the genes of Streptomyces hygroscopicus'
- GENE. INTERNATIONAL SYMPOSIUM ON BIOLOGY OF ACTINOMYCETES, 11-16 AUGUST, 1991. Bd. 115 , 1992 , AMSTERDAM NL Seiten 127 - 132 WOHLLEBEN, W., ET AL. 'Identification and characterization of phosphinothricin-tripeptide biosynthetic genes in Streptomyces viridochromogenes'
- EMBL SEQUENCE DATABASE ACC. NO. X65195 10-4-1992
- J. BACTERIOLOGY Bd. 173, Nr. 14 , Juli 1991 Seiten 4454 - 4463 RAIBAUD, A., ET AL. 'Nucleotide sequence analysis reveals linked N-acetyl hydrolase, thioesterase, transport, and regulatory genes encoded by the biosynthetic gene cluster of Streptomyces hygroscopicus'
- GENE Bd. 70 , 1988 , AMSTERDAM NL Seiten 25 - 37 WOHLLEBEN, W., ET AL. 'Nucleotide sequence of the phosphinothricin N-acetyltransferase gene from Streptomyces viridochromogenes Tü494 and its expression in Nicotina tabacum'

## Beschreibung

Die Erfindung betrifft die Verwendung von Deacetylasegenen, insbesondere zur Erzeugung transgener Pflanzen unter Einsatz gewebespezifischer Promotoren. In diesen Pflanzen kann die Entwicklung bestimmter Pflanzenteile gezielt verhindert werden.

Phosphinothricin (PTC, 2-Amino-4-Amino-4-methylphosphinobuttersäure) ist ein Glutaminsynthetase(GS)-lnhibitor. PTC ist ein "Baustein" des Antibiotikums Phosphinothricyl-Alanyl-Alanin. Dieses Tripeptid (PTT) ist aktiv gegen Gram-positive und Gram-negative Bakterien und auch gegen den Pilz Botrytis cinerea. PTT wird von dem Stamm Streptomyces viridochromogenes Tü494 produziert, der bei der Deutschen Sammlung für Mikroorganismen unter den Nummern DSM 40736 und DSM 4112 hinterlegt und erhältlich ist.

Aus der Deutschen Patentschrift 2 717 440 ist bekannt, daß PTC als Totalherbizid wirkt. In der veröffentlichten Anmeldung (EP-A-0257542) ist beschrieben, wie man mit Hilfe eines Phosphinothricin-N-Acetyltansferase(pat)-Gens Herbizid-resistente Pflanzen herstellt. Die von dem pat-Gen kodierte Phosphinothricin-N-Acetyltransferase modifiziert das intrazellulär auftretende PTC und detoxifiziert das Herbizid.

Die vorliegende Erfindung beschreibt nun Deacetylasegene (dea), deren Expressionsprodukte intrazellulär N-Acetyl-Phosphinothricin (N-Ac-PTC) bzw. N-Ac-PTT deacetylieren können und so wieder antibiotisch aktiv machen.

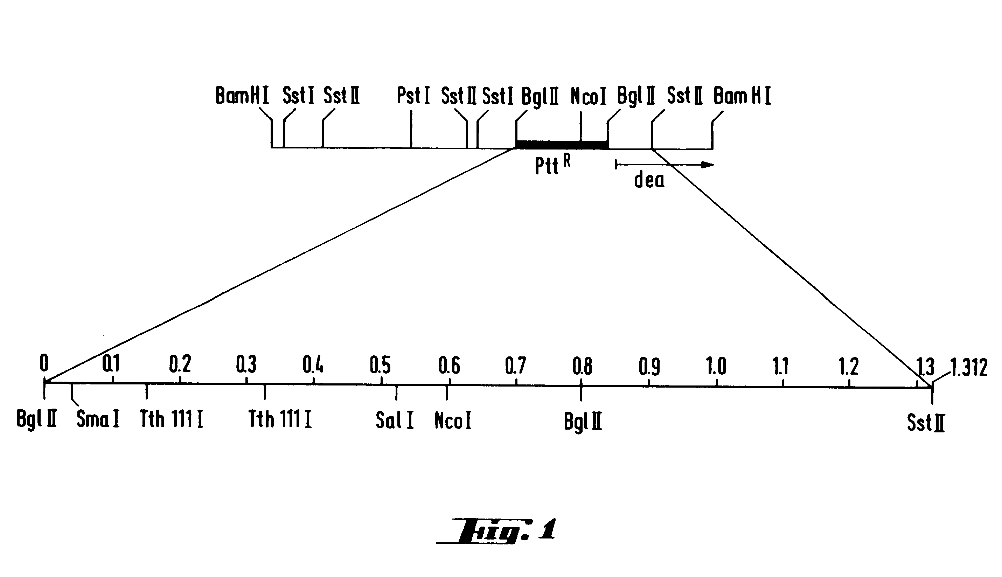

Ein erfindungsgemäßes N-Acetyl-Phosphinothricin-Tripeptid-Deacetylasegen läßt sich aus S. viridochromogenes Tü494 isolieren. Auf dem bereits bekannten 4.0-kb BamHI-Fragment (EP-A-0 257 542) liegt stromabwärts vom pat-Gen das dea-Gen. Dieses Gen liegt auf einem BgIII-BamHI-Fragment und ist durch die Sequenz genau bestimmt (Fig. 1 und Tab. 1). Die Proteinsequenz ist durch die DNA-Sequenz definiert. Als Translationsstartkodon dient ein ATG-Kodon, das in Bakterien und in Pflanzen erkannt wird; die Shine-Dalgarno-Sequenz ist durch Unterstreichen hervorgehoben. Dieses Gen kodiert in der PTT-Biosynthese den letzten Schritt, die Deacetylierung von inaktivem N-Acetyl-Phosphinothricin-Tripeptid zum aktiven PTT.

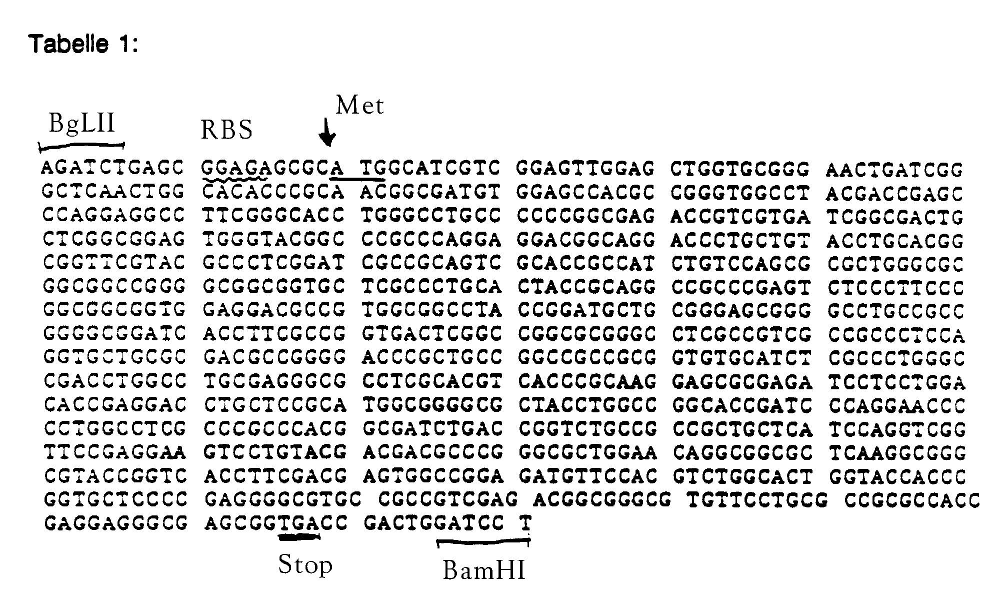

Von vielen Enzymen ist bekannt, daß ihre Spezifität nicht auf ein Substrat begrenzt ist. So dient die vom pat-Gen kodierte Phosphinothricin-N-Acetyltransferase in der PTT-Biosynthese eigentlich zur Acetylierung von Desmethyl-PTC und kann aufgrund ihrer Unspeziftät zur Detoxifizierung von PTC verwendet werden. Durch Überexpression des dea-Gens (mit Hilfe geeigneter Promotoren oder durch Klonierung auf high-copy-Vektoren) kann eine nicht hinreichend spezifische N-Acetyl-PTT-Deacetylase nun zur Aktivierung von N-Acetyl-Phosphinothricin eingesetzt werden.

Ein weiteres dea-Gen läßt sich aus E. coli gewinnen. Es wurde nämlich gefunden, daß sich in E. coli - im Gegensatz zu anderen Bakterien (z. B. Rhizobien und Streptomyceten) - nach Klonierung des pat-Gens in geeignete Expressionsvektoren (Strauch et al., Gene, 63, 65-74, 1988; Wohlleben et al., Gene, 70, 25-37, 1988) im sogenannten PAT-Assay (Doktorarbeit Inge Broer, Fakultät für Biologie der Universität Bielefeld, Expression des Phosphinthricin-N-Acetyltransferase-Gens aus Streptomyces viridochromogenes in Nicotiana tabacum, S. 42-43, 1989) keine Aktivität nachweisen läßt. Außerdem ist das pat-Gen in niedriger Kopienzahl in E. coli nicht in der Lage, PTT-Resistenz zu verleihen, da die endogene Deacetylase die Wirkung der Phosphinothricin-N-Acetyltransferase aufhebt. Schließlich kann diese Deacetylase-Aktivität durch die effektive Hemmung der GS-Aktivität nach Zugabe von N-Acetyl-Phosphinothricin direkt nachgewiesen werden. N-Ac-PTC wird durch die Deacetylase zu PTC umgesetzt, das dann in bekannter Weise die GS hemmt, was sich im γ-Glutamyl-Transferase-Assay (Bender et al., J. Bacteriol. 129, 1001-1009, 1977) messen läßt. Dies liegt an einer endogenen Deacetylase-Aktivität von E. coli.

Diese Aktivität sollte nicht in der argE-Mutante, die literaturbekannt ist, zu finden sein (Baumberg, Molec. Gen. Genetics 106, 162-173, 1970). Weitere E. coli Deacetylase-Mutanten sind leicht selektionierbar: Nach klassischer (Delic et al., Mut. Res. 9, 167-182, 1970; Drake und Baltz, Ann. Rev. Biochem. 45, 11-38, 1976) bzw. Tn5-Mutagenese (Kleckner, Ann. Rev. Genet. 15, 341-404, 1981) lassen sich auf mit PTT supplementiertem Minimalmedium solche Mutanten dadurch erkennen, daß nur sie nach Transformation mit einem in einen Niedrigkopienzahlvektor klonierten pat-Gen wachsen können.

Damit läßt sich das Deacetylasegen aus E. coli durch Anlegen einer Genbank in z. B. der E. coli argE-Mutante bzw. in einer neu isolierten Mutante mit herkömmlichen Verfahren (Maniatis et al., Molecular Cloning: a Laboratory Manual, Cold Spring Harbor Laboratory, Cold Spring Harbor, New York, 1982) isolieren.

Verfahren zur Isolierung weiterer Deacetylasegene ergeben sich aus dem oben Beschriebenen: Z. B. Isolierung neuer Organismen, die trotz Anwesenheit eines pat-Gens auf einem Niedrigkopienzahlvektor PTT-sensitiv sind, und anschließende Isolierung eines Deacetylasegens.

pat- und dea-Gene können in einem weiteren Aspekt der Erfindung zusammen mit gewebespezifischen Promotoren eingesetzt werden, um die Entwicklung bestimmter Pflanzengewebe gezielt zu verhindern. Eine spezielle Anwendung ist z. B. die Herstellung männlich steriler Pflanzen.

Die Herstellung von Hybridsaatgut in der Pflanzenzüchtung ist davon abhängig, eine Selbstbefruchtung der Mutterpflanze mit großer Sicherheit zu vermeiden. In der Natur kommen für viele Pflanzenarten männlich sterile Mutanten vor, die in der Züchtung eingesetzt werden. Der molekulare Mechanismus der cytoplasmatischen männlichen Sterilität (cms) ist bis heute nicht vollständig geklärt. Auch gibt es für viele Kultursorten, wie z. B. Beta vulgaris keine cms-Variante. Es ist daher von großem Interesse für die Landwirtschaft, auf molekulargenetischem Wege definierte cms-Mutanten aller wichtigen Kultursorten zu erzeugen. Die Firma PGS/Belgien hat in der Patentanmeldung PCT/EP 89/00495 eine solche Methode vorgestellt. Sie beruht auf der Zerstörung des die Pollenmutterzellen umgebenden Gewebes (Tapetum). Zu diesem Zweck wird ein RNAse-Gen mit einem Tapetum-spezifischen Promotor (Mariani et al.; Nature 347, 737-741, 1990) fusioniert. Die ausschließliche Expression des Gens in den Tapetumzellen sorgt für die selektive Zerstörung des Gewebes und verhindert damit die Bildung reifen Pollens. Eine Pflanze, die dieses Gen trägt, sollte nur nach Fremdbefruchtung Samen bilden können. Ein wesentlicher Nachteil dieses Systems ist die Tatsache, daß Nachkommen dieser Pflanze ebenfalls männlich steril sind und daher im Feld, wo sie auf Selbstbefruchtung angewiesen sind, keine Samen bilden können. Dies gelingt nur dann, wenn der männliche Partner der Kreuzung ein Gen trägt, das die Wirkung der RNAse in den Nachkommen aufheben kann. Dies soll nach der oben genannten offengelegten Patentanmeldung durch das barstar-Gen erfolgen. Tatsache hierbei ist, daß nur genetisch veränderte, das heißt transgene Partner in der Kreuzung genutzt werden können.

Nachstehend sind Verfahren zur Herstellung von cms-Pflanzen vorgestellt, die es zulassen, transgene Mutterpflanzen mit beliebigen artgleichen Partnern zu kreuzen. Dies wird durch Kombination eines dea-Gens unter Kontrolle z.B. eines Tapetum-Promotors in Verbindung mit einem konstitutiv exprimierten pat-Gen erreicht. Durch Applikation von PTC bzw. PTT wird gezielt die Glutaminsynthethase in den Tapetumzellen gehemmt und diese zum Absterben gebracht. Ein noch einfacheres System besteht in der Herstellung transgener Pflanzen, die lediglich ein einziges Fremd-Gen enthalten, nämlich ein dea-Gen unter Kontrolle eines gewebespezifischen, hier Tapetum-Promotors, sowie Applikation von N-Ac-PTC bzw. N-Ac-PTT auf die Pflanze.

Verallgemeinert umfaßt die Erfindung folglich zur gewebespezifischen Inhibierung mit Hilfe eines Deacetylasegens, vorzugsweise vorgenannten dea-Gens aus E. coli oder S. viridochromogenes Tü 494 folgende Verfahren:
1) Durch Pat-Aktivität PTT bzw. PTC-resistente Pflanzen (z. B. erzeugt wie in EP 0257542 beschrieben) werden mit dem Deacetylasegen aus Streptomyceten unter Kontrolle eines in Pflanzen gewebespezifischen Promotors transformiert. Nach Applikation von PTT oder PTC führt die Expression des Deacetylasegens zur Aufhebung der Phosphinothricin-N-Acetyltransferase-Aktivität in den entsprechenden Geweben. Diese werden dann selektiv abgetötet, während die restliche Pflanze resistent ist.
2) PTT- bzw. PTC-resistente Pflanzen werden mit dem E. coli Deacetylasegen unter Kontrolle eines gewebespezifischen Promotors transformiert. Nach Applikation von PTT oder PTC führt die Expression des Deacetylasegens zur Aufhebung der Phosphinothricin-N-Acetyltransferase-Aktivität in den entsprechenden Geweben. Diese werden dann selektiv abgetötet, während die restliche Pflanze resistent ist.
   Durch Verwendung von N-Acetyl-Phosphinothricin bzw. N-Acetyl-Phosphinothricin-Tripeptid kann dieses System vereinfacht werden. Beide Substanzen sind nicht als Herbizid aktiv, werden aber von Pflanzen aufgenommen, transportiert und nicht sofort abgebaut. Eine Deacetylaseaktivität für N-Acetyl-Phosphinothricin und N-Acetyl-Phosphinothricin-Tripeptid ist bisher in Pflanzen nicht nachgewiesen. So läßt sich das oben beschriebene 2-Gen-System auf ein 1-Gen-System reduzieren und damit entscheidend vereinfachen, wie unten weiter ausgeführt:
3) Beliebige Pflanzen werden mit einem Streptomyceten-Deacetylasegen unter Kontrolle eines gewebespezifischen Promotors transformiert. Nach Applikation von N-Acetyl-Phosphinothricin oder N-Acetyl-Phosphinothricin-Tripeptid führt die gewebespezifische Expression zum sofortigen Absterben des entsprechenden Gewebes.
4) Beliebige Pflanzen werden mit einem Deacetylasegen aus E.coli unter Kontrolle 3eines gewebespezifischen Promotors transformiert. Nach Applikation von N-Acetyl-Phosphinothricin oder N-Acetyl-Phosphinothricin-Tripeptid führt die gewebespezifische Expression zum sofortigen Absterben des entsprechenden Gewebes.

Wegen der höheren Spezifität der Streptomyceten-Deacetylase für N-Acetyl-Phosphinothricin-Tripeptid wird man im Fall 3) vorzugsweise N-Acetyl-Phosphinothricin-Tripeptid, im Fall 4) N-Acetyl-Phosphinothricin einsetzen, wenn man hohe Aktivitäten benötigt. Als gewebespezifische Promotoren können alle beschriebenen Promotoren Verwendung finden, deren selektive Expression in bestimmten Geweben nachgewiesen ist (z. B. Koltunow et al., The Plant Cell., Vol. 2, 1201-1224, 1990). Natürlich sind auch alle neu isolierten Promotoren mit ähnlichen Eigenschaften geeignet. Außer gewebespezifischen Promotoren können auch solche Promotoren eingesetzt werden, die einer anderen Art der Regulation (z. B. zeitlich, streßbedingt, umweltabhängig) unterworfen sind und die gewebespezifisch auftritt.

Diese Verfahren ermöglichen des weiteren die Analyse der Differenzierung der Zellregulation sowie die Erzeugung von Pflanzen, in denen die Entwicklung bestimmter Pflanzenteile gezielt verhindert wurde, vorzugsweise die Herstellung männlich steriler Pflanzen.

Eine weitere Anwendung ist der Einsatz eines dea-Gens zur Identifizierung selektiv exprimierter Promotoren. Kloniert man DNA-Fragmente mit Promotoraktivität vor dea-Gene, so zeigt der selektive Ausfall von Gewebeteilen nach Applikation von N-Acetyl-Phosphinothricin bzw. N-Acetyl-Phosphinothricin-Tripeptid die Spezifität des Promotors an.

Die Erfindung betrifft schließlich positive Selektionssysteme. Entweder in Kombination mit dem pat-Gen und PTT (bzw. PTC) zusammen mit einem dea-Gen oder mit N-Acetyl-Phosphinothricin (bzw. N-Acetyl-Phosphinothricin-Tripeptid) und einem dea-Gen allein können die Zellen selektioniert werden, in denen das dea-Gen inaktiviert wurde. Damit lassen sich erfolgreiche Klonierung (Insertionsinaktivierung) aber auch seltene Ereignisse (z. B. Transposition) direkt selektionieren. Weitere Aspekte der Erfindung sind in den Beispielen aufgeführt.

### Beispiel 1: Fusion des Deacetylasekodierbereichs mit eukaryontischen Transkriptionssignalen

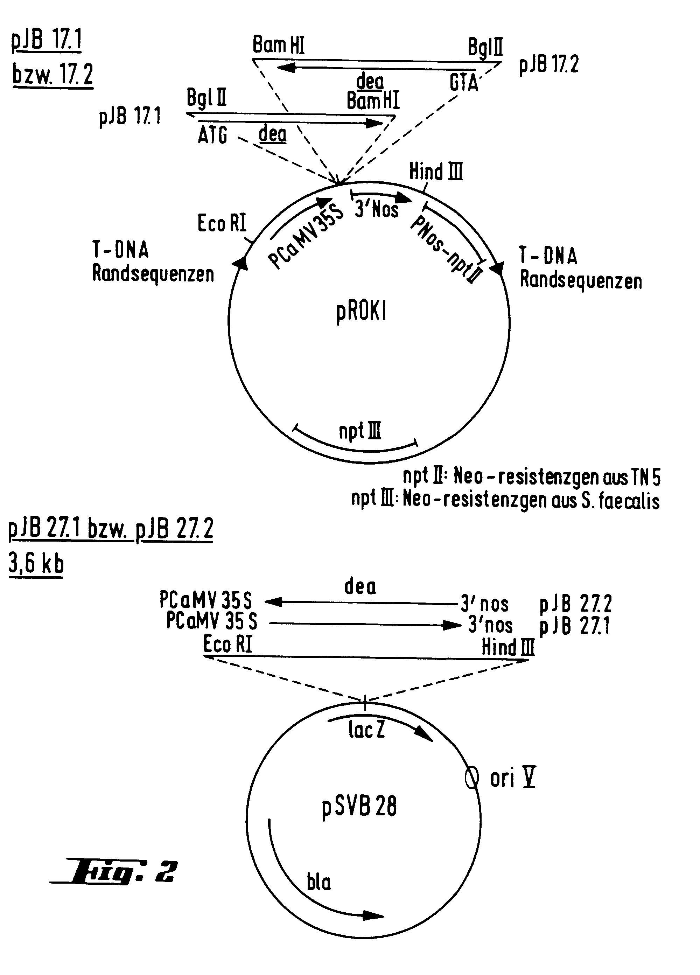

Aus einem E. coli Stamm wurde das Plasmid pPRI (siehe EP-0 257 542) isoliert und mit BamHI und BgIII gespalten. Die verdaute DNA wurde in einem Agarosegel aufgetrennt und ein 0.9 kb Fragment aus dem Gel isoliert. Der Vektor pROKI (Baulcombe et al., Nature 321, 446-449, 1986) wurde ebenfalls mit BamHI restringiert. Die beiden Ansätze wurden vereinigt und ligiert. Das Ligationsgemisch wurde nach E. coli S17.1 (Simon et al., Bio/Technology 1,784-791, 1983) transformiert. Auf kanamycinhaltigen Medien wachsende Kolonien wurden auf Nitrozellulosefilter übertragen und nach 12h Inkubation bei 37°C lysiert. Die DNA der Bakterien wurde auf dem Filter fixiert. das aus dem Agarosegel isolierte 0,9kb Fragment wurde durch Inkubation bei 100°C einzelsträngig gemacht. Anschließend wurde der fehlende Strang mit Klenowpolymerase und Digoxigenin markierten Nukleotiden aufsynthetisiert. Der markierte Strang wurde als Probe zur Hybridisierung mit der auf den Filter gebundenen bakteriellen DNA genutzt. Hybridisierende Klone ließen sich mit Hilfe einer Antikörperreaktion nachweisen. Die DNA der positiven Klone wurde mittels Qiagen-Lyse isoliert und mit BamHI/EcoRI sowie BamHI/HindIII verdaut. Diese Restriktion ermöglicht die Bestimmung der Orientierung des inserierten 0.9kb Fragmentes. Das Plasmid mit der Orientierung I wurde als pIB17.1, das mit der Orientierung II als pIB17.2 bezeichnet (siehe Fig. 2).

### Beispiel 2: Nachweis der Deacetylierung von N-Acetyl-PTC und N-Acetyl-PTT durch das Deacetylasegen

Es konnte gezeigt werden, daß die in dem Vektor pROKI klonierten eukaryontischen Transkriptionssignale auch eine Expression in R. meliloti, A. tumefaciens und E. coli ermöglichen.

Die Plasmide pIB17.1 und pIB17.2 wurden daher mittels 2-Faktor-Kreuzung in den Rhizobium meliloti Stamm 2011 transferriert. Durch Inkubation von R. meliloti Wildtypstämmen mit radioaktiv markiertem N-Acetyl-PTC konnte gezeigt werden, daß dieser Stamm N-Acetyl-PTC nicht deacetyliert. (Nach Inkubation von pIB17.1 tragenden Stämmen mit N-Acetyl-PTC und N-Acetyl-PTT kann die Deacetylierung mittels Dünnschichtchromatographie nachgewiesen werden). Es konnte ebenfalls gezeigt werden, das R. meliloti sehr sensitiv auf PTC und PTT reagiert. Daher läßt sich die Deacetylierung auch über die Hemmung der R. meliloti Glutaminsynthetasen durch das freigesetzte PTC nachweisen.

### Beispiel 3: Transfer des modifizierten Deacetylasegens in Nicotiana tabacum

Das in Beispiel 1 modifizierte Deacetylasegen wurde mittels einer 2-Faktor Kreuzung nach A. tumefaciens LBA4404 transferiert. Mit den so entstandenen Stämmen LBA4404/17.1 und LBA4404/17.2 wurden Nicotiana tabacum Blattscheiben inkubiert und nach 3 Tagen auf ein Kanamycin haltiges Sprossinduktionsmedium umgesetzt. Regenerierende kanamycinresistente Sprosse können durch Southern-hybridisierung auf die Anwesenheit des Deacetylasegens getestet werden. Nach Behandlung mit N-Acetyl-PTC oder N-Acetyl-PTT werden dann die Pflanzen durch das freigesetzte PTC bzw. PTT abgetötet.

### Beispiel 4: Konstruktion eines Vektors zur transienten Expression des modifizierten Deacetylasegens in E. coli und Tabakprotoplasten

Das modifizierte Deacetylasegen aus pIB17.1 und pIB17.2 wurde durch EcoRI/HindIII Verdauung aus den Plasmiden herausgeschnitten. Die restringierte DNA wurde im Agarosegel aufgetrennt und jeweils ein 0,9 kb Fragment isoliert. Der Vektor pSVB28 (Arnold und Pühler, Gene 70, 171-179, 1988) wurde ebenfalls mit EcoRI/HindIII verdaut. Die beiden Ansätze wurden vereinigt und ligiert. Nach Transformation in den β-Galactosidase-negativen E. coli Stamm JM83 zeigten alle Vektor tragenden Klone eine Blaufärbung, während Klone, die einen Vektor mit Insertion des Deacetylasegens tragen, weiß blieben. Aus den so identifizierten Klonen wurde die DNA isoliert und mit EcoRI/HindIII verdaut. Anhand des Restriktionsmusters ließen sich die Klone mit dem modifizierten Deacetylasegen erkennen. Die konstruierten Vektoren tragen die Bezeichnung pIB27.1 und pIB27.2 (siehe Fig. 2). Sie liegen in E. coli mit großer Kopienzahl vor.

### Beispiel 5: Transiente Expression des modifizierten Deacetylasegens in Tabakprotoplasten

Aus den in Beispiel 4 konstruierten E. coli Stämmen wurde die Plasmid-DNA isoliert. Junge Tabakblätter wurden mit Verdauungsenzymen für 20h inkubiert. Die aus dem Blattgerippe fallenden Protoplasten wurden gereinigt und in einem Transferpuffer mit Polyethylenglycol (PEG) und der isolierten DNA inkubiert. Anschließend wurden die Protoplasten gewaschen und in einer Kultufflüssigkeit (K3-Medium) aufgenommen. Nach 3 Tagen Inkubation bei schwacher Beleuchtung wurden die regenerierenden Protoplasten aufgeschlossen und die Rohextrakte mit radioaktiv markiertem N-Acetyl-PTC und N-Acetyl-PTT inkubiert. Das deacetylierte PTC bzw. PTT läßt sich über Dünnschichtchromatographie nachweisen.

### Beispiel 6: Verfahren zur Erzeugung männlich steriler Kulturpflanzen unter Verwendung des Deacetylasegens aus S. viridochromogenes unter Kontrolle eines tapetumspezifischen Promotors.

Das Deacetylasegen aus Streptomyces viridochromogenes wird mit einem tapetumspezifischen Promotor aus Nicotiana tabacum fusioniert und über Agrobakterien vermittelte Blattscheiben-Transformation in Tabakzellen eingebracht. Die aus diesen Zellen regenerierenden Pflanzen werden zu einem beliebigen Zeitpunkt vor der Blüte mit N-Acetyl-PTC oder N-Acetyl-PTT gespritzt. Es kann gezeigt werden, daß N-Acetyl-PTC in der Pflanzenzelle stabil ist und in alle Zellen transportiert wird. Keine der beiden Substanzen hat erkennbare negative Folgen für die Wildtyppflanze. Sobald sich die ersten Tapetumzellen bilden, beginnen sie mit der Expression des Deacetylasegens. Das in der Zelle gespeicherte N-Acetyl-PTC oder N-Acetyl-PTT wird durch das Enzym deacetyliert und damit in seine wirksame Form überführt. Es hemmt die Glutaminsynthetase der Zellen und führt so zu einem schnellen Absterben. Reife Pollen können nicht mehr entstehen. Zusätzlich ist auch die Bildung der Deacetylase unterbrochen. Umliegende Zellen sollten nicht beeinträchtigt werden. Wird die Pflanze nicht mit N-Acetyl-PTC oder N-Acetyl-PTT behandelt, ist sie voll fertil. Damit erübrigt sich eine Aufhebung der cms durch ein Gen des männlichen Partners der Kreuzung. Gleichzeitig liegt eine genau definierte Mutation vor, die ohne Auswirkungen auf die Wüchsigkeit und Nutzbarkeit der Pflanze bleibt.

### Beispiel 7: Identifizierung von gewebespezifischen Promotoren in transgenen Pflanzen

Mit Hilfe des Deacetylasegens aus Streptomyces viridochromogenes lassen sich gewebespezifische Promotoren direkt in der Pflanze identifizieren.

Das Deacetylasegen wird ohne einen Promotor so an das rechte oder linke Ende einer entwaffneten T-DNA kloniert, daß ein an der Insertionsstelle der T-DNA befindlicher Promotor im Pflanzengenom in das Gen hineinlesen und damit zu seiner Expression führen kann. Transgene Pflanzen werden über Stecklingsvermehrung geklont. Ein Klon wird mit N-Acetyl-PTC oder N-Acetyl-PTT behandelt und auf eventuell absterbende Gewebe hin untersucht. Mittels reverser PCR kann das getroffene Gen aus einem nicht mit N-Acetyl-PTC oder N-Acetyl-PTT behandelten Klon vermehrt und isoliert werden (Kahl und Weising, Gentransfer bei Pflanzen, Biologie in unserer Zeit, Nr. 6, s. 181, 1988).

### Beispiel 8: Nachweis von N-Acetyl-Phosphinothricin (PPT) - Deacetylase-Aktivität in Bodenproben

Je 500 mg Bodenproben (sandiger Lehm, Schwanheimer Düne) wurden auf 40 % der maximalen Wasserkapazität eingestellt und mit 5 µl einer 15 mM Stammlösung von ¹⁴[C]-L-N-Acetyl-PPT versetzt. Die Testproben wurden für verschiedene Zeiten (0 Stunden, 4, 7, 11 und 14 Tage) bei 28°C inkubiert und anschließend durch Extraktion mit 1 x 500 µl und 1 x 250 µl Wasser aufgearbeitet. Von den vereinigten wäßrigen Überständen wurden je 14 µl auf Dünnschichtchromatographie-Platten (HPTLC-Cellulose von Merck) aufgetragen und 2 x in n-Propanol: 25 % Ammoniak = 3 : 2 als Laufmittel entwickelt. Die Versuche wurden durch Autoradiographie ausgewertet. N-Acetyl-PPT und PPT konnten durch Vergleich der R₁-Werte der radioaktiven Spots mit den entsprechenden Referenzsubstanzen identifiziert werden. Es zeigte sich, daß
N-Acetyl-PPT im Boden innerhalb von 14 Tagen fast vollständig zu PPT metabolisiert wird. In einem Kontrollversuch mit sterilen Bodenproben (Boden 4 Stunden bei 200°C) erwies sich die Substanz dagegen als vollständig stabil.

### Beispiel 9: Isolierung und Identifizierung von Bodenmikroorganismen mit einer N-Acetyl-PPT-spezifischen Deacetylase-Aktivität

Je 1 g Boden wurde mit 10 ml 10 mM NaCl, 10 mM NaPhosphat-Puffer, pH = 7,0 für 1 Stunde bei Raumtemperatur extrahiert. Zur Selektion verschiedener Gruppen von Mikroorganismen wurden die Bodenüberstände auf die folgenden Agar-Medien ausplattiert, sowie zum Animpfen von Anreicherungskulturen in Erlenmeyerkolben mit den entsprechenden Flüssigmedien verwendet:
(1) MS1-Medium (für Eubakterien):
   5 mM Glucose
   5 mM Succinat
   10 mM Glycerin
   1 g/l NH₄Cl
   50 ml/l Lösung A
   25 ml/l Lösung B

   - Lösung A:: 50 g/l K₂HPO₄
   - Lösung B:: 2,5 g/l MgSO₄
   0,5 g/l NaCl
   25 ml/l Spurenelemente
(2) Chitin-Medium (für Actino- und Streptomyceten, sowie chitinovore Bakterien):
   10 g/l Krabbenchitin
   1 g/l (NH₄)₂SO₄
   0,5 g/l MgSO₄
   50 ml/l Lösung A
   1 ml/l Spurenelemente
(3) Antibiotika-Medium (für höhere Pilze):
   20 g/l Malzextrakt
   10 g/l Glucose
   2 g/l Hefeextrakt
   0,5 g/l (NH₄)₂SO₄
   50 /ug/ml Tetracyclin

Alle Medien enthielten 5 mM N-Acetyl-PPT. Die Agar-Platten und die Flüssigkulturen wurden für 3-5 Tage bei 28°C inkubiert.

Von den Selektiv-Agar-Platten wurden je 20 Einzelkolonien isoliert und in 5 ml Flüssigkulturen mit dem entsprechenden Medium überimpft. Nach 3-5 Tagen Wachstum wurden die Zellen bei 10 000 rpm abzentrifugiert und die Überstände im Aminosäureanalysator (Biotronic LC 5001) auf Bildung von PPT untersucht. Aus der Selektion mit Chitin-Medium konnte auf diese Weise eine PPT-positive Kultur (CB 10) isoliert weden.
Die Deacetylase-Aktivität der Zellen dieser Kultur wurde anschließend noch durch Biotransformation mit ¹⁴[C]-L-N-Acetyl-PPT als Substrat überprüft. Dazu wurden 1,5 ml der Kultur wie oben abzentrifugiert, das Zellpellet 1 x in 10 mM NaCl, 10 mM NaPhosphat-Puffer, pH = 7,0 gewaschen und in 100 µl des gleichen Puffers resuspeniert. 10 µl dieser Suspension wurden mit 10 µl einer 0,25 mM Lösung von ¹⁴[C]-L-N-Acetyl-PT versetzt und für 15 Stunden bei 28°C inkubiert. Dann wurden die Bakterien abzentrifugiert und 7 µl des Überstandes durch Dünnschichtchromatographie und Autoradiographie wie in Beispiel 1 beschrieben analysiert. Es konnte eine nahezu quantitative Umsetzung von N-Acetyl-PPT in PPT festgestellt werden. Der Versuch zeigt außerdem, daß die gefundene Deacetylase das L-Enantiomer des acetylierten PPT als Substrat akzeptiert.
Zur weiteren Reinigung der Stämme mit der gewünschten Deacetylase-Aktivität wurde die Kultur CB 10 auf LB-Agar (10 g/l Trypton, 5 g/l Hefe-Extrakt, 10 g/l NaCl, 15 g/l Agar) ausplattiert und für 2 Tage bei 28°C bebrütet. Von der Platte wurden 10 Einzelkoloniern isoliert, in Chitin-Flüssigmedium überimpft und die Kulturen wie oben beschrieben auf N-Acetyl-PPT-Deacetylase-Aktivität getestet. Die Deacetylase-positiven Isolate wurden erneut ausplattiert, um die Einheitlichkeit der Kultur zu überprüfen. Der Stamm mit der höchsten Deacetylase-Aktivität wurde als Xanthomonas maltophilia identifiziert (DSM Hinterlegungs-Nr. DSM 7192).

Die Anreicherungskulturen der Bodenproben in den verschiedenen Flüssigmedien wurden wie oben beschrieben auf Deacetylierung von N-Acetyl-PPT getestet. Dabei erwiesen sich nur die Chitin-Medium-Kulturen als Deacetylase-positiv. Nach Ausplattierung auf Chitin-Agar wurden von diesen Kulturen insgesamt 40 Einzelkolonien isoliert, in Chitin-Flüssigmedium angezogen und anschließend auf Deacetylase-Aktivität untersucht. Dabei wurden sechs positive Isolate gefunden (BoK1, BoK5, BoK9, BCÜ 1, BCÜ2, BCÜ3), aus denen durch nochmaliges Ausstreichen auf Agar-Platten und Weiterkultivieren von Einzelkolonien (siehe oben) die aktiven Reinkulturen gewonnen werden konnten. Der Stamm mit der höchsten Deacetylase-Aktivität wurde als Microbacterium imperiale identifiziert (DSM-Hinterlegungs-Nr. DSM 7191).

### Beispiel 10: N-Acetyl-PPT-Deacetylase-Enzymtests mit den isolierten Mikroorganismen

Von den Stämmen BoK1 und BoK5 wurden 5 ml Vorkulturen in LB-Medium über Nacht bei 28°C angezogen und davon je 0,5 ml in 20 ml LB-Medium bzw. 20 ml Chitin-Medium mit 1 mM N-Acetyl-PPT überimpft. Die LB-Kulturen wurden für 15 Stunden, die Chitin-Kulturen für 4 Tage in 100 ml Erlenmeyer-Kolben bei 28°C und 150 rpm inkubiert. Anschließend wurden die Zellen durch Zentrifugation für 10 min. bei 10 000 rpm geerntet, die Zellpellets 1 x in 10 ml mM NaCl, 10 mM NaPhosphat-Puffer, pH = 7,0 gewaschen, ausgewogen und auf c = 100 mg/ml in 100 mM Tris/HCl, pH = 8,0 resuspendiert. Die Suspensionen wurden mit 1 Volumen 100 mM N-Acetyl-PPT gemischt und in 50 ml Erlenmeyer-Kolben für 24 Stunden bei 28°C und 220 rpm inkubiert. Nach dem Abtrennen der Zellen durch Zentrifugation für 10 min bei 5000 rpm wurde der PPT-Gehalt in den Überständen im Aminosäureanalysator bestimmt (siehe Beispiel 9). Die Ergebnisse sind in der Tabelle 2 zusammengefaßt.

**Tabelle 2**

| N-Acetyl-PPT-Deacetylase-Tests mit Bodenmikroorganismen | | | |
|---|---|---|---|
| Stamm: | Medium: | Konzentration an PPT im Überstand | |
| | | [mM]: | [%]*: |
| BoK1 | LB | 0,7 | 2,7 |
| BoK1 | Chitin | 13,9 | 55,5 |
| BoK5 | LB | 6,0 | 23,9 |
| BoK5 | Chitin | 14,3 | 57,2 |

| | | | |
|---|---|---|---|
| *: bezogen auf das L-Enantiomer im N-Acetyl-PPT-Racemat. | | | |

### Beispiel 11: N-Acetyl-PPT-Deacetylase-Enzymtests mit Actinomyceten

Bei den beiden Actinomyceten-Stämmen Actinoplanes liguriae (IFO Nr. 13997) und Actinoplanes sp. (Stammsammmlung Zentralforschung Nr. A 1015) wurden bei Fermentationsversuchen in Anwesenheit von N-Acetyl-PPT und durch Biotransformation mit ¹⁴[C]-L-N-Acetyl-PPT als Substrat ebenfalls eine N-Acetyl-PPT-spezifische Deacetylase-Aktivitäten gefunden.

Zur Bestimmung der Umsatzraten wurden mit den beiden Stämmen Biotransformationen wie in Beispiel 3 beschrieben durchgeführt. Dabei wurden die folgenden Anzuchtmedien verwendet:
- Medium A:: 0,2 % Hefe-Extrakt
0,2 % Fleischextrakt
0,4 % Polypepton (aus Sojamehl)
1 % Glucose
- Medium B:: 20 g/l Haferflocken
1 ml/l Spurenelemente

Die Ergebnisse sind in der Tabelle 3 zusammengefaßt.

**Tabelle 3**

| N-Acetyl-PPT-Deacetylase-Tests mit Actinomyceten | | | |
|---|---|---|---|
| Stamm: | Medium: | Konzentration an PPT im Überstand | |
| | | [mM]: | [%]*: |
| Actinoplanes liguriae (IFO Nr. 13997) | A | 3,3 | 13,2 |
| Actinoplanes liguriae (IFO Nr. 13997) | B | 7,6 | 30,4 |
| Actinoplanes sp. (Nr. A 1015) | A | 11,0 | 44,0 |
| Actinoplanes sp. (Nr. A 1015) | B | 2,7 | 10,8 |

| | | | |
|---|---|---|---|
| *: bezogen auf das L-Enantiomer im N-Acetyl-PPT-Racemat | | | |

Weitere Bodenisolate mit N-Acetyl-PPT-spezifischer Deacetylase-Aktivität:
- Aus der Kultur CB 10:: Clavibacter michiganense insidiosum
Agrobacterium tumefaciens
Agrobacterium oxydans
Bacillus amyloliquefaciens
Bacillus macerans
- Aus der Kultur BoK1:: Alcaligenes faecalis
Escherichia coli
- Aus der Kultur BoK5:: Staphylococcus hominis
- Aus der Kultur BCÜ1:: Micrococcus luteus A
Acinetobacter johnsonii
Microbacterium laeraniformans
- Aus der Kultur BCÜ2:: Acinetobacter calcoaceticus

## Patentansprüche

1. Verfahren zur Herstellung transgener Pflanzen mit selektiv zerstörbaren Pflanzenteilen, dadurch gekennzeichnet, daß man in die Pflanze ein Deacetylasegen unter Kontrolle eines gewebespezifischen Promoters bringt und die betreffenden Gewebeteile durch geeignete rechtzeitige Behandlung mit N-Acetyl-PTC oder N-Acetyl-PTT zum Absterben bringt.

2. Verfahren zur Herstellung transgener Pflanzen mit selektiv zerstörbaren Pflanzenteilen, dadurch gekennzeichnet, daß die Pflanze eine PTC-Resistenz besitzt und zusätzlich ein Deacetylasegen unter Kontrolle eines gewebespezifischen Promoters erhält und die betreffenden Gewebeteile durch geeignete rechtzeitige Behandlung mit PTC oder PTT zum Absterben bringt.

3. Verfahren nach Anspruch 1 oder 2, dadurch gekennzeichnet, daß das Deacetylasegen aus E.coli stammt und die Pflanze mit N-Acetyl-PTC bzw. PTC behandelt wird.

4. Verfahren nach Anspruch 1 oder 2, dadurch gekennzeichnet, daß das Deacetylasegen aus Streptomyces stammt und die Pflanze mit N-Acetyl-PTT bzw. PTT behandelt wird.

5. Verfahren nach einem oder mehreren der Ansprüche 1 - 4, dadurch gekennzeichnet, daß männlich sterile Pflanzen erzeugt werden.

6. Verfahren nach einem oder mehreren der Ansprüche 1 - 5, dadurch gekennzeichnet, daß das Deacetylasegen unter der Kontrolle des Tapetum-Promoters steht.

7. Verwendung eines Deacetylasegens zum gezielten Außer-Funktion-Setzen spezifischer Pflanzenteile, in dem das Deacetylasegen unter der Kontrolle eines gewebespezifischen Promoters exprimiert wird und die betreffenden Gewebeteile durch geeignete und rechtzeitige Behandlung mit N-Acetyl-PTC oder N-Acetyl-PTT selektiv abgetötet werden.

8. Verwendung eines Deacetylasegens zum gezielten Außer-Funktion-Setzen spezifischer Pflanzenteile, in dem das Deacetylasegen unter der Kontrolle eines gewebespezifischen Promoters exprimiert wird und die betreffenden Gewebeteile durch geeignete und rechtzeitige Behandlung mit PTC oder PTT selektiv abgetötet werden.

## Claims

1. A process for the production of transgenic plants with selectively destroyable parts, which comprises, in the plant, bringing a deacetylase gene under the control of a tissue-specific promoter and causing death of the tissue portions concerned by suitable timely treatment with N-acetyl-PTC or N-acetyl-PTT.

2. A process for the production of transgenic plants with selectively destroyable parts, which comprises the plant having PTC resistance and additionally obtaining a deacetylase gene under the control of a tissue-specific promoter and causing death of the tissue portions concerned by suitable timely treatment with PTC or PTT.

3. The process as claimed in claim 1 or 2, wherein the deacetylase gene originates from E. coli and the plant is treated with N-acetyl-PTC or with PTC.

4. The process as claimed in claim 1 or 2, wherein the deacetylase gene originates from Streptomyces and the plant is treated with N-acetyl-PTT or with PTT.

5. The process as claimed in one or more of claims 1-4, wherein male-sterile plants are produced.

6. The process as claimed in one or more of claims 1-5, wherein the deacetylase gene is under the control of the tapetum promoter.

7. The use of a deacetylase gene for the targeted prevention of specific parts of the plant from functioning, where the deacetylase gene is expressed under the control of a tissue-specific promoter and the tissue portions concerned are selectively destroyed by suitable and timely treatment with N-acetyl-PTC or N-acetyl-PTT.

8. The use of a deacetylase gene for the targeted prevention of specific parts of the plant from functioning, where the deacetylase gene is expressed under the control of a tissue-specific promoter and the tissue portions concerned are selectively destroyed by suitable and timely treatment with PTC or PTT.

## Revendications

1. Procédé pour la production de plantes transgéniques avec des parties de plante destructibles sélectivement, caractérisé en ce que l'on met un gène de désacétylase dans la plante sous contrôle d'un promoteur spécifique de tissus et on tue les parties de tissus par traitement approprié en temps utile avec la N-acétyl-PTC ou le N-acétyl-PTT.

2. Procédé pour la production de plantes transgéniques avec des parties de plante destructibles sélectivement, caractérisé en ce que la plante présente une résistance à la PTC et contient en plus un gène de désacétylase sous le contrôle d'un promoteur spécifique de tissus et l'on tue les parties de tissus concernées par traitement approprié en temps utile avec la PTC ou le PTT.

3. Procédé selon la revendication 1 ou 2, caractérisé en ce que le gène de désacétylase provient de E. *coli* et la plante est traitée par la N-acétyl-PTC ou par la PTC.

4. Procédé selon la revendication 1 ou 2, caractérisé en ce que le gène de désacétylase provient de *Streptomycètes* et la plante est traitée par le N-acétyl-PTT ou le PTT.

5. Procédé selon une ou plusieurs des revendications 1 à 4, caractérisé en ce que l'on produit des plantes à pollen stérile.

6. Procédé selon une ou plusieurs des revendications 1 à 5, caractérisé en ce que le gène de désacétylase est contrôlé par le promoteur de tapetum.

7. Utilisation d'un gène de désacétylase pour la mise hors fonction contrôlée des parties de plante spécifiques, dans laquelle le gène de désacétylase est exprimé sous le contrôle d'un promoteur spécifique de tissus et on tue sélectivement les parties de plante concernées par un traitement approprié et en temps utile avec la N-acétyl-PTC ou le N-acétyl-PTT.

8. Utilisation d'un gène de désacétylase pour la mise hors fonction contrôlée des parties de plante spécifiques dans lesquelles est exprimé le gène de désacétylase sous le contrôle d'un promoteur spécifique de tissus et les parties végétales concernées sont anéanties par un traitement approprié en temps utile avec PTC ou PTT.
